(19) Europäisches Patentamt
European Patent Office
Office européen des brevets

(11) Publication number : **0 228 172 B1**

(12) # EUROPEAN PATENT SPECIFICATION

(45) Date of publication of patent specification : **07.03.90**

(51) Int. Cl.$^5$ : **C 07 D311/22**, C 07 D335/06

(21) Application number : **86308857.1**

(22) Date of filing : **13.11.86**

(54) **Preparation of 6-substituted 4-chromanones.**

(30) Priority : **24.12.85 US 813195**

(43) Date of publication of application :
**08.07.87 Bulletin 87/28**

(45) Publication of the grant of the patent :
**07.03.90 Bulletin 90/10**

(84) Designated contracting states :
**AT BE CH DE ES FR GB IT LI NL SE**

(56) References cited :
**EP-A- 0 168 006**
**GB-A- 1 499 323**
**CHEMISCHE BERICHTE, VOL 80, 1947, Verlagchemie-GmbH Weinheim/Bergstr. und Berlin H.A.OFFE et al. "Ueber die Kondensation von Hydrochinon und Hydrochinon-Derivaten mit Carbonsäuren unter dem Einfluss von wasserfreier Flusssäure" pp 464-469**
**CHEMICAL ABSTRACTS , VOL 100, no.1, 1st Jan. 84, Columbus,Ohio,USA NIPPON ZOKI PHARMACEUTICAL CO. "Chromanone derivatives", pp.545, column 2, p. 546,column 1, abstract-no 6 340r**
**CHEMICAL ABSTRACTS, vol. 100, no.17, 23.April 1984, Columbus, Ohio, USA HOKKO CHEMICAL INDUSTRY CO. "Thiochroman-4-ones" page 636, column 1, abstract-no. 138 955e**

(73) Proprietor : **Mallinckrodt, Inc. (a Delaware corporation)**
**675 McDonnell Boulevard P.O. Box 5840**
**St. Louis Missouri 63134 (US)**

(72) Inventor : **Patton, Jerry R.**
**702 Westglen village Drive**
**Ballwin Missouri 63021 (US)**
Inventor : **Gurusamy, Narayanasamy**
**217 Steanboat Lane, Apt. 6**
**Ballwin Missouri 63011 (US)**

(74) Representative : **Eyles, Christopher Thomas**
**W.P. THOMPSON & CO. 50 Lincoln's Inn Fields**
**London WC2A 3PF (GB)**

**EP 0 228 172 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for preparing 6-substituted 4-chromanones from phenolic acrylate ester compounds derivable from para-substituted phenolic compounds and beta-unsubstituted acrylic acid compounds which are esterifiable therewith. The 6-substituted 4-chromanones prepared by the process of the invention are useful as intermediates for preparing pharmaceutical agents.

Preparation of 4-chromanones via condensation of beta-substituted, beta, beta-disubstituted and alpha, beta-disubstituted acrylic acids with phenolic compounds in anhydrous hydrogen fluoride is disclosed by Offe and Barkow in Chem. Ber. 80, 458 (1947), hereinafter Offe I. However, Offe I does not disclose such preparation using beta-unsubstituted acrylic acids. Offe and Barkow go on to discuss in Chem. Ber. 80, 464 (1947), hereinafter Offe II, the condensation of hydroquinone and hydroquinone derivatives with carboxylic acids under the influence of anhydrous hydrogen fluoride. Offe I indicates that it is possible but not practical to prepare 4-chromanones in hydrogen fluoride from phenol and beta-unsubstituted acrylic acid compounds such as acrylic acid per se. Offe I does not disclose how such preparation is possible. Similarly, Amakasu and Sato indicate in J. Org. Chem. 31, 1433 (1966) that 4-chromanones are not obtainable via reaction of acrylic acid or alpha-monosubstituted acrylic acid with phenols.

It has now been found unexpectedly that 6-substituted 4-chromanones more particularly defined hereinbelow can be prepared in practical and efficient manner from phenolic acrylate esters of para-substituted phenolic compounds and beta-unsubstituted acrylic acid compounds in the presence of hydrogen fluoride. The beta-unsubstituted acrylic acid compounds include acrylic acid per se and alpha-monosubstituted acrylic acid. It has also been found that the esters can be prepared in practical and efficient manner by condensing the phenolic compounds with the acrylic acid compounds in the presence of hydrogen fluoride in an initially anhydrous system. Isolation of the various intermediate compounds is not required, thereby advantageously permitting preparation of the 6-substituted chromanones from the phenolic compounds and acrylic acid compounds by an integral overall process in a single reaction mixture and a single reaction vessel.

Generally stated, the present invention provides a process for preparing a 6-substituted-4-chromanone having the formula

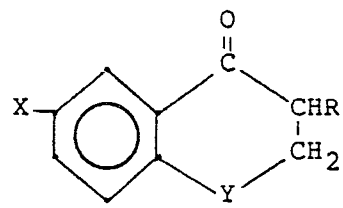

(1)

where X is selected from halogen atoms, nitro, amino and alkyl having from 1 to 20 carbon atoms ;

Y is an oxygen or sulfur atom ; and

R is selected from a hydrogen atom, halogen atoms, alkyl having from 1 to 20 carbon atoms and aryl having from 6 to 12 carbons ; characterised in that it includes the steps of :

(a) effecting rearrangement of a phenolic acrylate ester of a phenol or thiophenol, said ester having the formula

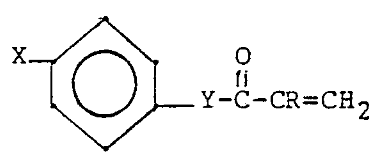

(2)

where X, Y and R are as defined above, to a (2-hydroxy- or -mercapto-phenyl)vinyl ketone having the formula

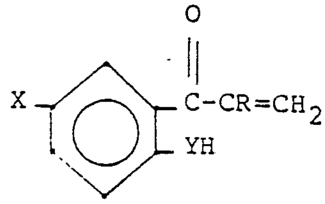

(3)

where X, Y and R are as defined above, by reaction at a temperature of up to 150 °C and at a pressure of

not more than 18.24 bar (250 psig) in a substantially anhydrous liquid reaction mixture containing not more than 5 % water by weight and initially comprising said phenolic acrylate ester and from 5 to 50 moles of hydrogen fluoride per mole of said phenolic acrylate ester ; and

(b) effecting cyclization of said ketone in the presence of from 5 to 50 moles of hydrogen fluoride per mole of said ketone to form the 6-substituted-4-chromanone at a temperature of up to 200 °C and at a pressure of not more than 18.24 bar (250 psig) in a substantially anhydrous liquid reaction mixture containing not more than 5 % water by weight and initially comprising said ketone and hydrogen fluoride.

In another aspect, generally stated, the above-described process of this invention further comprises preparing said phenolic acrylate ester by contacting a para-substituted phenolic compound having the formula

$$p—X—C_6H_4—Y—Z \qquad (4)$$

where X is selected from halogen atoms, nitro, amino and alkyl having from 1 to 20 carbon atoms ;

Y is an oxygen or sulfur atom ; and

Z is selected from a hydrogen atom, alkyl having from 1 to 20 carbon atoms and aryl having from 6 to 12 carbon atoms, with a beta-unsubstituted acrylic acid compound having the formula

$$CH_2{=}CR—C({=}O)OH \qquad (5)$$

where R is selected from a hydrogen atom, halogen atoms, alkyl having from 1 to 20 carbon atoms and aryl having from 6 to 12 carbons, in the presence of a condensation-effecting amount of hydrogen fluoride under ester-forming condensation conditions to form said phenolic acrylate ester, said condensation conditions comprising a substantially anhydrous liquid reaction mixture of said para-substituted phenolic compound, said beta-unsubstituted acrylic acid compound and hydrogen fluoride.

Hence according to another aspect of the invention there is provided a process for preparing a 6-substituted-4-chromanone having the formula (1) set out above characterised in that it includes the steps of :

(a) preparing a phenolic acrylate ester having the formula (2) set out above by contacting a para-substituted phenolic compound having the formula

$$p—X—C_6H_4—Y—Z \qquad (4)$$

where X and Y are as defined above ; and Z is selected from a hydrogen atom, alkyl having from 1 to 20 carbon atoms and aryl having from 6 to 12 carbon atoms, with a beta-unsubstituted acrylic acid compound having the formula

$$CH_2{=}CR—C({=}O)OH \qquad (5)$$

where R is as defined above ; in the presence of from 5 to 50 moles of hydrogen fluoride per mole of said para-substituted phenolic compound at a temperature of up to 150 °C and at a pressure of not more than 21.68 bar (300 psig) in a substantially anhydrous liquid reaction mixture containing not more than 5 % water by weight and initially comprising said para-substituted phenolic compound, said beta-unsubstituted acrylic acid compound and hydrogen fluoride ;

(b) effecting rearrangement of said phenolic acrylate ester having the formula (2) set out above to a (2-hydroxy- or -mercapto-phenyl) vinyl ketone having the formula (3) set out above by reaction at a temperature of up to 150 °C and at a pressure of not more than 18.24 bar (250 psig) in a substantially anhydrous liquid reaction mixture containing not more than 5 % water by weight and initially comprising said phenolic acrylate ester of formula (2) set out above and from 5 moles to 50 moles of hydrogen fluoride per mole of said phenolic acrylate ester of formula (2) set out above ; and

(c) effecting cyclization of said ketone in the presence of from 5 to 50 moles of hydrogen fluoride per mole of said ketone to form the 6-substituted-4-chromanone at a temperature of up to 200 °C and at a pressure of not more than 18.24 bar (250 psig) in a substantially anhydrous liquid reaction mixture containing not more than 5 % water by weight and initially comprising said ketone and hydrogen fluoride.

The phenolic acrylate ester of formula (2) above can be prepared in any suitable manner, including by well known methods such as transesterification of a phenolic ester containing the $p—X—C_6H_4—Y—$ moiety of formula (2) and an acid moiety having its carbonyl carbon bonded directly to the Y atom with the beta-unsubstituted acrylic acid compound of formula (5) above in the presence of a mineral acid. Another well known method is esterification of a phenolic compound of formula (4) above where Z is H with an acrylol chloride which is an acid chloride of an acid of formula (5).

Preferably, however, the ester of formula (2) is prepared by contacting a para-substituted phenolic compound of formula (4) above with a beta-unsubstituted acrylic acid compound of formula (5) above. Such contacting is effected in the presence of a condensation-effecting amount of hydrogen fluoride under ester-forming condensation conditions, including an initially substantially anhydrous liquid reaction mixture of the phenolic compound, the acrylic acid compound and hydrogen fluoride.

As used herein, the term « substantially anhydrous » means that the substance referred to does not contain more than 5 % water by weight, preferably not more than 1 % water.

The ester-forming reaction can be carried out at any suitable temperature, provided that the reaction mixture is under sufficient pressure to keep the para-substituted phenolic compound, the acrylic acid compound, the hydrogen fluoride, the ester and any other reaction mixture components which may be employed in the liquid state. In general, the temperature may be, for example, from 0 °C to 150 °C, preferably from 20 °C to 50 °C, and more preferably from 40 °C to 50 °C. In general, the reaction pressure may be, for example, from 1.01 bar to 21.68 bar (0 to 300 psig), preferably from 1.01 bar to 11.35 bar (0 to 150 psig), and more preferably not more than 7.90 bar (100 psig). The reaction can be carried out in any reaction vessel suitable for holding hydrogen fluoride and the other components of the reaction mixture. The reaction vessel is preferably a closed vessel equipped with means for stirring the reaction mixture, means for measuring the temperature of the mixture and means for controlling such temperature. Although the components of the reaction mixture may be added in any order, preferably the para-substituted phenolic compound and acrylic acid compound are admixed and thereafter the hydrogen fluoride is added to the resulting admixture with stirring.

The acrylic acid compound and the para-substituted phenolic compound may be added in stoichiometric amounts (i. e. in 1 : 1 mole ratio). Advantageously, the acrylic acid is added in excess of the stoichiometric amount, thereby tending to effect complete reaction of the phenolic compound. The mole ratio of the acrylic acid compound to the phenolic compound may be, for example, from 1 : 1 to 2 : 1, preferably from 1.05 : 1 to 1.5 : 1, and more preferably 1.5 : 1.

Hydrogen fluoride is added in any condensation-effective amount and may be added in an amount, for example, from 5 to 50 moles, preferably from 10 to 20 moles, and more preferably 15 moles, per mole of the phenolic compound added. The time required for carrying out the ester-forming reaction depends on the particular compounds and reaction conditions employed. In general, the reaction can be carried to completion within one hour and in many instances within 0.5 hour.

The ester-forming reaction is preferably carried out in the additional presence of an agent for inhibiting polymerization of the acrylic acid and the ester being prepared. Suitable polymerization-inhibiting agents include hydroquinone, sulfur, phenothiazine (preferred), compatible mixtures thereof and the like. The inhibitor can be included in any suitable amount effective for inhibiting polymerization of the acrylic acid compound and the ester being formed, such as, for example, from 0.01 to 0.1 mole per 100 moles of the acrylic acid compound added. On the same basis, approximately 0.1 mole of phenothiazine is preferred.

Water adversely interferes with the ester-forming reaction. For best results, the initial reaction mixture is free of water. Hydrogen fluoride serves to catalyze the reaction and tie up the water formed during the course of the ester-forming reaction.

Whether the phenolic acrylate ester is prepared by the ester-forming process of this invention or is otherwise provided, the ester is next converted to the chromanone of formula (1) above. Such conversion is carried out by first converting the ester to the (2-hydroxy- or mercapto-phenyl) vinyl ketone of formula (3) above and thereafter converting the ketone to the 6-substituted 4-chromanone of formula (1) above.

The first conversion, i. e. rearrangement of a phenolic acrylate ester of formula (2) above to a ketone of formula (3) above, is effected in the presence of a Fries rearrangement-effective amount of hydrogen fluoride under Fries rearrangement conditions, preferably in a substantially anhydrous liquid reaction mixture initially comprising the phenolic acrylate ester compound and hydrogen fluoride. Although a substantially anhydrous reaction mixture is not required for the rearrangement reaction, generally better yields result therefrom.

The ester-to-ketone rearrangement reaction can be carried out at any suitable temperature, provided that the reaction mixture is under sufficient pressure to keep the reaction mixture components in the liquid state. In general, the temperature may be, for example, from 0 °C to 150 °C, preferably from 100 °C to 120 °C, and more preferably 100 °C. In general, the reaction pressure may be, for example, from 4.46 bar to 18.24 bar (50 to 250 psig), preferably from 7.90 bar to 11.35 bar (100 to 150 psig), and more preferably 10.66 bar (140 psig). The reaction can be carried out in any reaction vessel suitable for holding hydrogen fluoride and the balance of the reaction mixture. The reaction vessel is preferably a closed vessel equipped with means for stirring the reaction mixture, means for measuring the temperature of the mixture and means for controlling such temperature. Where the ester is not prepared in hydrogen fluoride, the components of the rearrangement reaction mixture may be added in any order, but preferably the hydrogen fluoride is added to the ester with stirring. Advantageously and preferably, the ester is prepared in the presence of hydrogen fluoride as herein taught and rearrangement to the ketone is effected in the reaction mixture employed in the ester-preparation step without isolating the resulting ester.

Hydrogen fluoride is employed in any rearrangement-effective amount and may be present in an amount, for example, from 5 to 50 moles, preferably from 10 to 20 moles, and more preferably 15 moles, per mole of the ester. The time required for carrying out the ester-to-ketone rearrangement reaction depends on the particular ester being rearranged and reaction conditions employed. In general, the reaction can be carried to completion within two hours and in many instances within about 0.1 to 0.5 hour.

The acrylate ester-rearrangement reaction is preferably carried out in the additional presence of an agent for inhibiting polymerization of the ester (and the vinyl ketone being prepared). Suitable polymerization-inhibiting agents include hydroquinone, sulfur, phenothiazine (preferred), compatible mixtures thereof and the like. The inhibitor can be included in any suitable amount effective for inhibiting polymerization of the acrylate ester compound and the ketone being prepared, such as, for example, from 0.01 to 0.1 mole per 100 moles of the acrylate ester compound employed. On the same basis, approximately 0.1 mole of phenothiazine is preferred.

Those skilled in the art will appreciate that, as shown by formula (3) above, in the ketone the carbonyl carbon atom of the vinyl carboxy group is directly attached to the benzene ring ortho to the hydroxy or mercapto group represented by —YH in such formula. Rearrangement to such ketone rather than rearrangement to a structure wherein such carbonyl carbon atom is attached meta to the —YH group is critical for preparing 6-substituted 4-chromanones. It is also critical that the X substituent be para to the —YH group. If the X substituent were ortho or meta to the —YH group, the ester would rearrange to a structure wherein the vinyl carboxy group is attached para to the —YH group. Such structure would be inoperable for cyclization to a chromanone structure. Moreover, if X were ortho to the —YH group, only one of the two ortho positions would be available for displacement of a hydrogen atom in the ester by the vinyl carboxy group to form a 4-chromanone-precursor ketone.

After rearrangement of the ester to the ketone of formula (3), the ketone is cyclized to the corresponding 6-substituted 4-chromanone. Cyclization is effected in the presence of a cyclization-effective amount of hydrogen fluoride under cyclization conditions, preferably in a substantially anhydrous liquid reaction mixture initially comprising the ketone and hydrogen fluoride. Although a substantially anhydrous reaction mixture is not required for the cyclization reaction, generally better yields result therefrom.

The cyclization reaction can be carried out at any suitable temperature, provided that the reaction mixture is under sufficient pressure to keep the reaction mixture components in the liquid state. In general, the temperature may be, for example, from 100 °C to 200 °C, preferably from 100 °C to 120 °C, and more preferably 100 °C. In general, the reaction pressure may be, for example, from 4.46 bar to 18.24 bar (50 to 250 psig), preferably from 7.90 bar to 11.35 bar (100 to 150 psig), and more preferably 10.66 bar (140 psig). The reaction can be carried out in any reaction vessel suitable for holding hydrogen fluoride and the balance of the reaction mixture. The reaction vessel is preferably a closed vessel equipped with means for stirring the reaction mixture, means for measuring the temperature of the mixture and means for controlling such temperature. Where the ketone is not cyclized in the reaction mixture in which it was prepared, the components of the cyclization reaction mixture may be added in any order, but preferably the hydrogen fluoride is added to the ketone with stirring. Advantageously and preferably, cyclization of the ketone is effected in the reaction mixture employed in the ester-to-ketone rearrangement step without isolating the resulting ketone.

Hydrogen fluoride is employed in any cyclization-effective amount and may be present in an amount, for example, from 5 to 50 moles, preferably from 10 to 20 moles, and more preferably 15 moles, per mole of the ketone. The time required for carrying out the cyclization reaction depends on the particular ketone being cyclized and reaction conditions employed. In general, the reaction can be carried to completion within one hour.

The ketone-cyclization reaction is preferably carried out in the additional presence of an agent for inhibiting polymerization of the vinyl ketone. Suitable polymerization-inhibiting agents include hydroquinone, sulfur, phenothiazine (preferred), compatible mixtures thereof and the like. The inhibitor can be included in any suitable amount effective for inhibiting polymerization of the vinyl ketone, such as, for example, from 0.01 to 0.1 mole per 100 moles of the vinyl ketone employed. On the same basis, approximately 0.1 mole of phenothiazine is preferred.

The overall time for effecting rearrangement and cyclization is generally 3 to 6 hours.

After the cyclization reaction is complete, hydrogen fluoride can be recovered, if desired, by distillation of at least a portion thereof from the reaction mixture. With or without prior removal of hydrogen fluoride, the reaction mixture may be admixed with water to precipitate the 6-substituted 4-chromanone, which then may be washed and dried if desired. Preferably, cold water, e. g. from 0 °C to 20 °C (preferably 0 °C to 5 °C) is admixed with the reaction mixture.

Illustrative of the 6-substituted 4-chromanones included within formula (1) above are :

(a) 6-fluoro-4-chromanone,
(b) 6-chloro-4-chromanone,
(c) 6-bromo-4-chromanone,
(d) 6-methyl-4-chromanone,
(e) 6-nitro-4-chromanone,
(f) 6-amino-4-chromanone,
(g) the 6-substituted 3-methyl-4-chromanones corresponding to the above compounds (a) through (f),
(h) the 6-substituted 3-halo-4-chromanones corresponding to the above compounds (a) through (f), and

(i) the 6-substituted thio-4-chromanones corresponding to the above compounds (a) through (h).

Illustrative of the corresponding para-substituted phenolic compounds included within formula (4) above and from which the above illustrative 6-substituted 4-chromanones may be prepared are :

(a′) p-fluorophenol,
(b′) p-chlorophenol,
(c′) p-bromophenol,
(d′) p-methylphenol (i. e. p-cresol),
(e′) p-nitrophenol,
(f′) p-aminophenol,
(g′) same as compounds (a′) through (f′),
(h′) see (g′), and
(i′) the p-thiophenols corresponding to the above compounds (a′) through (h′).

Illustrative of the corresponding beta-unsubstituted acrylic acid compounds included within formula (5) above and from which the above illustrative 6-substituted 4-chromanones may be prepared are :

(a″) acrylic acid for the above compounds (a) through (f) and their corresponding thio compounds
(b″) methacrylic acid for the above compounds (g) and their corresponding thio compounds, and
(c″) 2-halo-acrylic acid for the above compounds (h) and their corresponding thio compounds.

Although there is no known limit on the halogens which may be employed as R and X in the above formulas, fluorine, chlorine, bromine and iodine atoms are generally preferred, and more preferably F, Cl, and Br.

Generally preferred alkyl and aryl groups from which R and Z in the above formulas can be selected are methyl, ethyl and phenyl.

Practice of the present invention is illustrated by the following non-limiting examples. All parts, percents and other amounts throughout this disclosure are by weight unless otherwise indicated.

In the examples which follow, all products were characterized as 4-Chromanones by melting point, HPLC, infrared analysis and NMR.

### Example 1

### Preparation of 6-Fluoro-4-Chromanone

A 400 ml stainless steel Parr reactor was charged with para-fluorophenol (28.4 grams), acrylic acid (28.8 grams), and an inhibitor phenothiazine (0.02 grams). The system was sealed, evacuated and cooled in dry ice/acetone before the anhydrous hydrogen fluoride (117 grams) was condensed into the system. The mixture was then warmed to 40 °C for one hour thirty minutes with stirring before being heated to 100 °C for four hours thirty minutes. The excess HF was then removed and the system cooled to 0 °C and opened to give 62 grams of brown oil which was washed with D.I. water to give 50 grams of material to be extracted. The 6-Fluoro-4-Chromanone was collected and recrystallized from methanol/water. The HPLC yield was 75 %, isolated 52 %.

### Example 2

### Preparation of 6-Fluoro-4-Chromanone

A 400 ml stainless steel Parr reactor was charged with previously prepared p-fluorophenylacrylate (41.5 grams) and phenothiazine (0.02 grams) before being sealed and evacuated. The system was cooled in dry ice/acetone and anhydrous hydrogen fluoride (40 grams) was vacuum transferred into the reactor. The mixture was heated to 100 °C for four hours before the hydrogen fluoride was vented and the system cooled to 0 °C. The reactor was opened and worked up as in Example 1 to give the desired 6-Fluoro-4-Chromanone.

### Example 3

### Preparation of 6-Nitro-4-Chromanone

Following the same procedure as Example 1, p-nitrophenol (34.8 grams), acrylic acid (27 grams), and an inhibitor, phenothiazine (0.02 grams) was added to the pressure reactor. The anhydrous hydrogen fluoride (110 grams) was added before the system was warmed. When the reaction temperature was attained, a pressure of 10.66 bar (140 psig) was noted, similar to previous reactions. Work-up was carried out as previous examples and the 6-Nitro-4-Chromanone was isolated and characterized.

Example 4

Preparation of 6-Bromo-4-Chromanone

Following the procedure of Example 1, parabromophenol (43.25 grams), acrylic acid (27 grams) and phenothiazine (0.02 grams) were added to the pressure reactor. The system was closed and evacuated before being cooled to — 78 °C and the anhydrous hydrogen fluoride (110 grams) was added. After the mixture was heated and stirred for six hours at 100 °C, 10.66 bar (140 psig), the excess hydrogen fluoride was vented and the reaction mixture cooled to 0-5 °C. After washing the mixture with D.I. water, HPLC analysis indicated one major product which was separated and characterized as 6-Bromo-4-Chromanone.

Example 5

Preparation of 6-Fluoro-3-Methyl-4-Chromanone

Using the procedure established in Example 1, the Parr reactor was charged with para-fluorophenol (28 grams), methacrylic acid (32 grams), and phenothiazine (0.02 grams). The system was sealed, evacuated, and cooled to — 78 °C before the anhydrous HF (110 grams) was vacuum transferred to the reactor. The mixture was warmed to 40 °C for one hour then up to 100 °C for five hours. The pressure was recorded at 12.04 bar (160 psig) at the reaction temperature. The excess hydrogen fluoride was vented and the mixture cooled to 5 °C before being washed with D.I. water. Analysis by HPLC indicated one major product which was isolated and characterized to be 6-Fluoro-3-Methyl-4-Chromanone. The presence of a minor amount (not more than 5 %) of residual hydroxy vinyl ketone corresponding to 6-fluoro-3-methyl-4-chromanone was evidenced by IR and HPLC analyses taken with the starting materials and preparation procedure.

Example 6

Preparation of 6-Fluoro-thio-4-Chromanone

The 400 ml Parr pressure reactor was charged with para-fluorothiophenol (19.2 grams), acrylic acid (16.2 grams), and phenothiazine (0.02 gram). The reactor was closed, evacuated and cooled to — 78 °C before the anhydrous hydrogen fluoride (70 grams) was added. The mixture was warmed to 40 °C for one hour then heated to 100 °C for 4.5 hours. After the reaction time had been reached, the excess hydrogen fluoride was vented and the product washed with D.I. water. Analysis by HPLC indicated the desired product, 6-Fluoro-thio-4-Chromanone to be present. Work-up gave the product which was characterized by infrared and NMR analysis.

Example 7

Preparation of 6-Fluoro-4-Chromanone

Using the procedure established in Example 1, the reactor was charged with p-fluoroanisole (31.5 grams), acrylic acid (27 grams) and phenothiazine (0.02 grams) before being sealed, evacuated, and cooled to — 78 °C. The anhydrous hydrogen fluoride (110 grams) was vacuum transferred into the reactor and the mixture warmed to 40 °C for one hour then 100 °C for 4.5 hours. The excess hydrogen fluoride was then vented and the reactor cooled to 5 °C before being opened and the contents washed with D.I. water. Analysis by HPLC indicated the presence of para-fluoroanisole and 6-Fluoro-4-Chromanone. The para-fluoroanisole was removed by distillation and the desired 6-Fluoro-4-Chromanone isolated by extraction and characterized.

Example 8

Attempted Preparation of 7-Fluoro-4-Chromanone

Following the same procedure established in Example 1, the reactor was charged with meta-fluorophenol (28 grams), acrylic acid (27 grams), and phenothiazine (0.02 grams). The reactor was sealed, evacuated and cooled to — 78 °C before the anhydrous hydrogen fluoride (125 grams) was added. The mixture was stirred and warmed to 40 °C for one hour then to 100 °C for 4.5 hours as the pressure was recorded at 10.66 bar (140 psig). After venting the excess hydrogen fluoride the reactor was cooled to 5 °C and the mixture washed with D.I. water. Analysis by HPLC indicated no 4-Chromanones to be present. A mixture of by-products were seen.

## Claims

1. A process for preparing a 6-substituted-4-chromanone having the formula

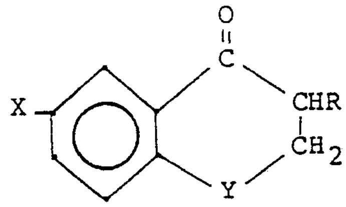

(1)

where X is selected from halogen atoms, nitro, amino and alkyl having from 1 to 20 carbon atoms ;

Y is an oxygen or sulfur atom ; and

R is a member selected from a hydrogen atom, halogen atoms, alkyl having from 1 to 20 carbon atoms and aryl having from 6 to 12 carbons ;

characterised in that it includes the steps of :

(a) effecting rearrangement of a phenolic acrylate ester of a phenol or thiophenol, said ester having the formula

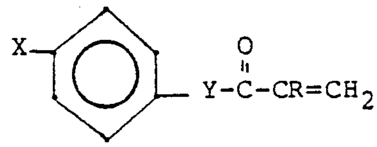

(2)

where X, Y and R are as defined above, to a (2-hydroxy- or -mercapto-phenyl) vinyl ketone having the formula

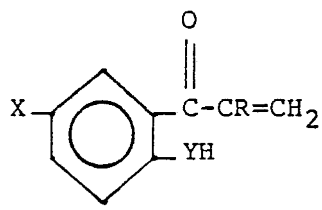

(3)

where X, Y and R are as defined above, by reaction at a temperature of up to 150 °C and at a pressure of not more than 18.24 bar (250 psig) in a substantially anhydrous liquid reaction mixture containing not more than 5 % water by weight and initially comprising said phenolic acrylate ester and from 5 to 50 moles of hydrogen fluoride per mole of said phenolic acrylate ester ; and

(b) effecting cyclization of said ketone in the presence of from 5 to 50 moles of hydrogen fluoride per mole of said ketone to form the 6-substituted-4-chromanone at a temperature of up to 200 °C and at a pressure of not more than 18.24 bar (250 psig) in a substantially anhydrous liquid reaction mixture containing not more than 5 % water by weight and initially comprising said ketone and hydrogen fluoride.

2. A process according to claim 1, characterised in that the cyclization step is effected in the reaction mixture employed in the rearrangement step without isolating said ketone.

3. A process according to claim 1 or claim 2, characterised in that the rearrangement and cyclization steps are effected in the presence of from 0.01 to 0.1 mole per 100 moles of said phenolic acrylate ester of an agent for inhibiting polymerization of said ester and said ketone.

4. A process for preparing a 6-substituted-4-chromanone having the formula (1) set out in claim 1 ; characterised in that it includes the steps of :

(a) preparing a phenolic acrylate ester having the formula (2) set out in claim 1 by contacting a para-substituted phenolic compound having the formula

$$p\text{—}X\text{—}C_6H_4\text{—}Y\text{—}Z \qquad (4)$$

where X and Y are as defined in claim 1 ; and Z is selected from a hydrogen atom, alkyl having from 1 to 20 carbon atoms and aryl having from 6 to 12 carbon atoms, with a beta-unsubstituted acrylic acid compound having the formula

$$CH_2{=}CR\text{—}C({=}O)OH \qquad (5)$$

where R is as defined in claim 1 ; in the presence of from 5 to 50 moles of hydrogen fluoride per mole of

said para-substituted phenolic compound at a temperature of up to 150 °C and at a pressure of not more than 21.68 bar (300 psig) in a substantially anhydrous liquid reaction mixture containing not more than 5 % water by weight and initially comprising said para-substituted phenolic compound, said beta-unsubstituted acrylic acid compound and hydrogen fluoride ;

(b) effecting rearrangement of said phenolic acrylate ester having the formula (2) set out in claim 1 to a (2-hydroxy- or -mercapto-phenyl) vinyl ketone having the formula (3) set out in claim 1 by reaction at a temperature of up to 150 °C and at a pressure of not more than 18.24 bar (250 psig) in a substantially anhydrous liquid reaction mixture containing not more than 5 % water by weight and initially comprising said phenolic acrylate ester of formula (2) and from 5 moles to 50 moles of hydrogen fluoride per mole of said phenolic acrylate ester of formula (2) ; and

(c) effecting cyclization of said ketone in the presence of from 5 to 50 moles of hydrogen fluoride per mole of said ketone to form the 6-substituted-4-chromanone at a temperature of up to 200 °C and at a pressure of not more than 18.24 bar (250 psig) in a substantially anhydrous liquid reaction mixture containing not more than 5 % water by weight and initially comprising said ketone and hydrogen fluoride.

5. A process according to claim 4, characterised in that the rearrangement step is effected in the reaction mixture employed in the ester-preparation step without isolating said ester.

6. A process according to claim 4 or claim 5, characterised in that the cyclization step is effected in the reaction mixture employed in the rearrangement step without isolating said ketone, whereby the 6-substituted-4-chromanone can be prepared from said para-substituted phenolic compound and said beta-unsubstituted acrylic acid compound in sequential steps performed in a single reaction mixture in a single reaction vessel.

7. A process according to any one of claims 4 to 6, characterised in that the ester-forming, rearrangement and cyclization steps are effected in the presence of from 0.01 to 0.1 mole per 100 moles of said acrylic acid compound of an agent for inhibiting polymerization of said acrylic acid compound, said ester and said ketone.

8. A process according to any one of claims 4 to 7, characterised in that said para-substituted phenolic compound is selected from p-fluorophenol, p-chlorophenol, p-bromophenol, p-cresol, p-nitrophenol, p-aminophenol and their corresponding p-thiophenols.

9. A process according to any one of claims 4 to 8, characterised in that said beta-unsubstituted acrylic acid compound is selected from acrylic acid, methacrylic acid and the 2-halo-acrylic acids.

10. A process according to any one of the preceding claims, characterised in that said ester is para-fluorophenylacrylate and said 6-substituted-4-chromanone is 6-fluoro-4-chromanone.

## Patentansprüche

1. Verfahren zur Herstellung eines 6-substituierten 4-Chromanons der Formel

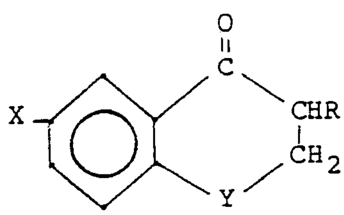

(1)

worin X unter Halogenatomen, Nitro, Amino und Alkyl mit 1 bis 20 Kohlenstoffatomen ausgewählt ist, Y ein Sauerstoff- oder Schwefelatom ist und R unter Wasserstoffatom, Halogenatomen, Alkyl mit 1 bis 20 Kohlenstoffatomen und Aryl mit 6 bis 12 Kohlenstoffatomen ausgewählt ist, gekennzeichnet durch die Stufen der

(a) Umlagerung eines phenolischen Acrylatesters eines Phenols oder Thiophenols mit der Formel

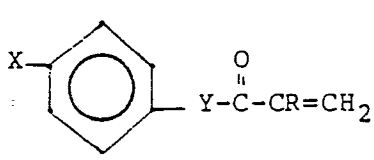

(2)

worin X, Y und R die oben definierten Bedeutungen haben, zu einem (2-Hydroxy- oder -Mercapto-phenyl) vinylketon der Formel

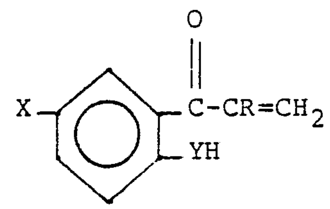

(3)

worin X, Y und R die oben definierten Bedeutungen haben, durch Umsetzung bei einer Temperatur bis zu 150 °C und einem Druck von nicht mehr als 18,24 bar in einem im wesentlichen wasserfreien flüssigen Reaktionsgemisch, das nicht mehr als 5 Gew.-% Wasser enthält und zu Beginn den phenolischen Acrylatester und 5 bis 50 Mole Fluorwasserstoff je Mol des phenolischen Acrylatesters aufweist, und

(b) Ringbildung des Ketons in Gegenwart von 5 bis 50 Molen Fluorwasserstoff je Mol Keton zu dem 6-substituierten 4-Chromanon bei einer Temperatur von bis zu 200 °C und bei einem Druck von nicht mehr als 18,24 bar in einem im wesentlichen wasserfreien, flüssigen Reaktionsgemisch, das nicht mehr als 5 Gew.-% Wasser enthält und zu Beginn das Keton und den Fluorwasserstoff aufweist.

2. Verfahren nach Anspruch 1, dadurch gekennzeichnet, daß die Ringbildung ohne Isolierung des Ketons in dem Reaktionsgemisch erfolgt, das in der Umlagerungsstufe benutzt wird.

3. Verfahren nach Anspruch 1 oder 2, dadurch gekennzeichnet, daß die Umlagerung und Ringbildung in Gegenwart von 0,01 bis 0,1 Mol je 100 Mole phenolischer Acrylatester eines Mittels zur Hemmung der Polymerisation des Esters und Ketons erfolgt.

4. Verfahren zur Herstellung eines 6-substituierten 4-Chromanons der in Anspruch 1 angegebenen Formel (1), gekennzeichnet durch die Stufen der

(a) Herstellung eines phenolischen Acrylatesters der in Anspruch 1 angegebenen Formel (2) durch Inberührungbringen einer para-substituierten phenolischen Verbindung der Formel

$$p—X—C_6H_4—Y—Z, \quad (4)$$

worin X und Y die in Anspruch 1 definierte Bedeutung haben und Z unter einem Wasserstoffatom, Alkyl mit 1 bis 20 Kohlenstoffatomen und Aryl mit 6 bis 12 Kohlenstoffatomen ausgewählt ist, mit einer beta-unsubstituierten Acrylsäure-Verbindung der Formel

$$CH_2=CR—C(=O)OH, \quad (5)$$

worin R die in Anspruch 1 definierte Bedeutung hat, in Gegenwart von 5 bis 50 Molen Fluorwasserstoff je Mol der para-substituierten phenolischen Verbindung bei einer Temperatur von bis zu 150 °C und bei einem Druck von nicht mehr als 21,68 bar in einem im wesentlichen wasserfreien flüssigen Reaktionsgemisch, das nicht mehr als 5 Gew.-% Wasser enthält und zu Beginn die para-substituierte phenolische Verbindung, die beta-unsubstituierte Acrylsäureverbindung und Fluorwasserstoff aufweist,

(b) Umlagerung des genannten phenolischen Acrylatesters der in Anspruch 1 angegebenen Formel (2) zu einem (2-Hydroxy- oder -Mercapto-phenyl) vinylketon der in Anspruch 1 angegebenen Formel (3) durch Umsetzung bei einer Temperatur von bis zu 150 °C und bei einem Druck von nicht mehr als 18,24 bar in einem im wesentlichen wasserfreien flüssigen Reaktionsgemisch, das nicht mehr als 5 Gew.-% Wasser enthält und zu Beginn den phenolischen Acrylatester der Formel (2) und 5 bis 50 Mole Fluorwasserstoff je Mol phenolischen Acrylatester der Formel (2) aufweist, und

(c) Ringbildung des Ketons in Gegenwart von 5 bis 50 Molen Fluorwasserstoff je Mol des Ketons unter Bildung des 6-substituierten 4-Chromanons bei einer Temperatur von bis zu 200 °C und bei einem Druck von nicht mehr als 18,24 bar in einem im wesentlichen wasserfreien flüssigen Reaktionsgemisch, das nicht mehr als 5 Gew.-% Wasser und zu Beginn das Keton und Fluorwasserstoff aufweist.

5. Verfahren nach Anspruch 4, dadurch gekennzeichnet, daß die Umlagerung ohne Isolierung des Esters in dem Reaktionsgemisch erfolgt, das bei der Esterherstellungsstufe benutzt wird.

6. Verfahren nach Anspruch 4 oder Anspruch 5, dadurch gekennzeichnet, daß die Ringbildung ohne Isolierung des Ketons in dem Reaktionsgemisch erfolgt, das in der Umlagerungsstufe benutzt wird, wodurch das 6-substituierte 4-Chromanon aus der para-substituierten phenolischen Verbindung und der beta-unsubstituierten Acrylsäure-Verbindung in aufeinanderfolgenden Stufen hergestellt werden kann, die in einem einzigen Reaktionsgemisch in einem einzigen Reaktionsbehälter durchgeführt werden.

7. Verfahren nach einem der Ansprüche 4 bis 6, dadurch gekennzeichnet, daß die Esterbildung, Umlagerung und Ringbildung in Gegenwart von 0,01 bis 0,1 Mol je 100 Mole Acrylsäure-Verbindung eines Mittels zur Hemmung der Polymerisation der Acrylsäure-Verbindung, des Esters und des Ketons durchgeführt werden.

8. Verfahren nach einem der Ansprüche 4 bis 7, dadurch gekennzeichnet, daß die para-substituierte phenolische Verbindung unter p-Fluorphenol, p-Chlorphenol, p-Bromphenol, p-Kresol, p-Nitrophenol, p-Aminophenol und ihren entsprechenden p-Thiophenolen ausgewählt wird.

9. Verfahren nach einem der Ansprüche 4 bis 8, dadurch gekennzeichnet, daß die beta-unsubstituierte Acrylsäure-Verbindung unter Acrylsäure, Methacrylsäure und den 2-Halogenacrylsäuren ausgewählt wird.

10. Verfahren nach einem der vorhergehenden Ansprüche, dadurch gekennzeichnet, daß der Ester para-Fluorphenylacrylat und das 6-substituierte 4-Chromanon 6-Fluor-4-chromanon ist.

**Revendications**

1. Procédé pour préparer une 4-chromannone 6-substituée répondant à la formule

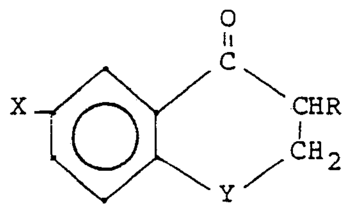

dans laquelle X est choisi parmi les atomes d'halogène, un nitro, un amino et un alkyle ayant de 1 à 20 atomes de carbone ;

Y est un atome d'oxygène ou de soufre ; et

R est choisi parmi un atome d'hydrogène, les atomes d'halogène, un alkyle ayant de 1 à 20 atomes de carbone et un aryle ayant de 6 à 12 atomes de carbone ;

caractérisé en ce qu'il comprend les stades consistant à :

(a) effectuer une transposition d'un ester acrylique phénolique d'un phénol ou thiophénol, ledit ester répondant à la formule

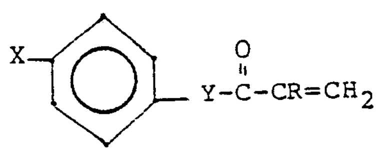

dans laquelle X, Y et R sont comme défini ci-dessus en une (2-hydroxy- ou 2-mercapto-phényl) vinylcétone répondant à la formule

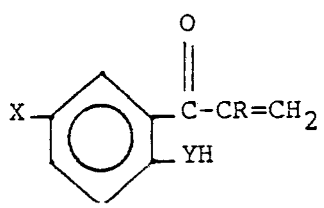

dans laquelle X, Y et R sont comme défini ci-dessus, par réaction à une température pouvant atteindre 150 °C et à une pression ne dépassant pas 18,24 bars (250 psig) dans un mélange réactionnel liquide essentiellement anhydre ne contenant pas plus de 5 % en poids d'eau et comprenant initialement ledit ester acrylique phénolique et de 5 à 50 moles de fluorure d'hydrogène par mole dudit ester acrylique phénolique ; et

(b) effectuer la cyclisation de ladite cétone, en présence de 5 à 50 moles de fluorure d'hydrogène par mole de ladite cétone, pour former la 4-chromannone 6-substituée, à une température pouvant atteindre 200 °C et à une pression ne dépassant pas 18,24 bars (250 psig) dans un mélange réactionnel liquide essentiellement anhydre ne contenant pas plus de 5 % en poids d'eau et comprenant initialement ladite cétone et du fluorure d'hydrogène.

2. Procédé selon la revendication 1, caractérisé en ce que le stade de cyclisation est effectué dans le mélange réactionnel utilisé dans le stade de transposition, sans isolement de ladite cétone.

3. Procédé selon la revendication 1 ou la revendication 2, caractérisé en ce que les stades de transposition et de cyclisation sont effectués en présence de 0,01 à 0,1 mole d'un agent inhibant la polymérisation dudit ester et de ladite cétone pour 100 moles dudit ester acrylique phénolique.

4. Procédé pour préparer une 4-chromannone 6-substituée répondant à la formule (1) comme indiqué dans la revendication 1, caractérisé en ce qu'il comprend les stades consistant à :

(a) préparer un ester acrylique phénolique répondant à la formule (2) indiquée dans la revendication 1 par contact d'un composé phénolique substitué en para répondant à la formule

$$p{-}X{-}C_6H_4{-}Y{-}Z \qquad (4)$$

dans laquelle X et Y sont comme défini dans la revendication 1 ; et Z est choisi parmi un atome d'hydrogène, un alkyle ayant de 1 à 20 atomes de carbone et un aryle ayant de 6 à 12 atomes de carbone, avec un acide acrylique non substitué en β répondant à la formule

$$CH_2{=}CR{-}C({=}O)OH \qquad (5)$$

dans laquelle R est comme défini dans la revendication 1 ; en présence de 5 à 50 moles de fluorure d'hydrogène par mole dudit composé phénolique substitué en para, à une température pouvant atteindre 150 °C et à une pression ne dépassant pas 21,68 bars (300 psig), dans un mélange réactionnel liquide essentiellement anhydre ne contenant pas plus de 5 % en poids d'eau et comprenant initialement ledit composé phénolique substitué en para, ledit acide acrylique non substitué en β et du fluorure d'hydrogène ;

(b) effectuer la transposition dudit ester acrylique phénolique de formule (2) indiquée dans la revendication 1 en une (2-hydroxy- ou 2-mercapto-phényl) vinylcétone répondant à la formule (3) indiquée dans la revendication 1 par réaction à une température pouvant atteindre 150 °C et à une pression ne dépassant pas 18,24 bars (250 psig), dans un mélange réactionnel liquide essentiellement anhydre ne contenant pas plus de 5 % en poids d'eau et comprenant initialement ledit ester acrylique phénolique de formule (2) et de 5 moles à 50 moles de fluorure d'hydrogène par mole dudit ester acrylique phénolique de formule (2) ; et

(c) effectuer la cyclisation de ladite cétone, en présence de 5 à 50 moles de fluorure d'hydrogène par mole de ladite cétone, pour former la 4-chromannone 6-substituée, à une température pouvant atteindre 200 °C et à une pression ne dépassant pas 18,24 bars (250 psig) dans un mélange réactionnel liquide essentiellement anhydre ne contenant pas plus de 5 % en poids d'eau et comprenant initialement ladite cétone et du fluorure d'hydrogène.

5. Procédé selon la revendication 4, caractérisé en ce que le stade de transposition est effectué dans le mélange réactionnel utilisé dans le stade de préparation de l'ester sans isolement dudit ester.

6. Procédé selon la revendication 4 ou la revendication 5, caractérisé en ce que le stade de cyclisation est effectué dans le mélange réactionnel utilisé dans le stade de transposition, sans isolement de ladite cétone, si bien que la 4-chromannone 6-substituée peut être préparée à partir dudit composé phénolique substitué en para et dudit acide acrylique non substitué en β selon des stades successifs effectués dans un mélange réactionnel unique dans un même réacteur.

7. Procédé selon l'une quelconque des revendications 4 à 6, caractérisé en ce que les stades de formation de l'ester, de transposition et de cyclisation sont effectués en présence de 0,01 à 0,1 mole d'un agent inhibant la polymérisation dudit composé acide acrylique, dudit ester et de ladite cétone pour 100 moles dudit composé acide acrylique.

8. Procédé selon l'une quelconque des revendications 4 à 7, caractérisé en ce que ledit composé phénolique substitué en para est choisi parmi le p-fluorophénol, le p-chlorophénol, le p-bromophénol, le p-crésol, le p-nitrophénol, le p-aminophénol et les p-thiophénols correspondants.

9. Procédé selon l'une quelconque des revendications 4 à 8, caractérisé en ce que ledit acide acrylique non substitué en β est choisi parmi l'acide acrylique, l'acide méthacrylique et les acides 2-halogénoacryliques.

10. Procédé selon l'une quelconque des revendications précédentes, caractérisé en ce que ledit ester est l'acrylate de para-fluorophényle et ladite 4-chromannone 6-substituée est la 6-fluoro-4-chromannone.